# EUROPEAN PATENT APPLICATION

(11) **EP 4 520 830 A1**
(43) Date of publication of application: **12.03.2025**
(21) Application number: 23816399.2
(22) Date of filing: 02.06.2023
(51) Int. Cl.: C12N 15/77, C12N 1/20, C07K 16/12, C12N 9/10, C12P 13/10

(54) **MICROORGANISM WITH L-ORNITHINE PRODUCTION CAPABILITY AND METHOD FOR PRODUCING L-ORNITHINE USING SAME**

(30) Priority: 02.06.2022 KR 20220067365
(71) Applicant: CJ CheilJedang Corporation, Seoul 04560 (KR)
(72) Inventor: PARK, Jinsub, Seoul 04560 (KR); BYUN, Hyo Jeong, Seoul 04560 (KR); LEE, Ji Yeon, Seoul 04560 (KR); HAN, Seunghee, Seoul 04560 (KR); KIM, Sang Jun, Seoul 04560 (KR)
(74) Representative: Meissner Bolte Partnerschaft mbB
(86) International application number: PCT/KR2023/007567
(87) International publication number: WO 2023/234739

(57) **Abstract**

Provided are a microorganism having L-ornithine-producing ability and a method of producing L-ornithine using the same.

## Description

### [Technical Field]

The present disclosure relates to a microorganism having L-ornithine-producing ability and a method of producing L-ornithine using the same.

### [Background Art]

L-amino acids are used in animal feeds, human medicines, and the cosmetics industries, and some L-amino acids are produced by fermentation using microorganisms. To improve the methods of producing L-amino acids using microorganisms, research using recombinant DNA technology has been conducted. For example, the L-amino acid production ability of microorganisms could be increased by expressing some genes that have been deleted or attenuated or by amplifying genes related to L-amino acid biosynthesis. In particular, improvement of the L-amino acid export ability of microorganisms has been considered as a key technology for increasing the L-amino acid production ability (US 10995378 B2). Therefore, it is be possible to greatly increase the production ability by releasing high concentrations of L-amino acids that are accumulated inside microorganisms by introducing and amplifying genes of which the L-amino acid export ability has been revealed.

### [Disclosure]

### [Technical Problem]

The present inventors have confirmed that when an exogenous LysE/ArgO family amino acid transporter protein is introduced into microorganisms, their L-ornithine-producing ability is improved, as compared to that of unmodified microorganisms, thereby completing the present disclosure.

### [Technical Solution]

An object of the present disclosure is to provide a recombinant microorganism of the genus *Corynebacterium* having L-ornithine-producing ability, the recombinant microorganism comprising a LysE/ArgO family amino acid transporter protein derived from a strain of the genus *Shewanella* or a polynucleotide encoding the LysE/ArgO family amino acid transporter protein.

Another object of the present disclosure is to provide a method of producing L-ornithine, the method comprising the step of culturing, in a medium, a recombinant microorganism of the genus *Corynebacterium* having L-ornithine-producing ability, the recombinant microorganism comprising a LysE/ArgO family amino acid transporter protein derived from a strain of the genus *Shewanella* or a polynucleotide encoding the LysE/ArgO family amino acid transporter protein.

### [Advantageous Effects]

A microorganism of the present disclosure into which an exogenous LysE/ArgO family amino acid transporter protein is introduced may have the increased L-ornithine-producing ability, as compared to existing unmodified microorganisms.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a recombinant microorganism of the genus *Corynebacterium* having L-ornithine-producing ability, the recombinant microorganism comprising a LysE/ArgO family amino acid transporter protein derived from a strain of the genus *Shewanella* or a polynucleotide encoding the LysE/ArgO family amino acid transporter protein.

As used herein, the term "LysE/ArgO family amino acid transporter" is a protein having LysE (lysine exporter) and/or ArgO (arginine exporter) functions belonging to the amino acid exporter family in bacteria. In other words, the LysE/ArgO family amino acid transporter refers to a protein having lysine and/or arginine export activity.

An amino acid sequence of the LysE/ArgO family amino acid transporter protein may be obtained from NCBI's Genbank which is a known database, etc.

For example, the LysE/ArgO family amino acid transporter protein of the present disclosure may be derived from a microorganism.

For another example, the LysE/ArgO family amino acid transporter protein of the present disclosure may be an exogenous protein that does not endogenously exist in microorganisms of the genus *Corynebacterium.*

For still another example, the LysE/ArgO family amino acid transporter protein of the present disclosure may be derived from a microorganism. The microorganism may be specifically derived from a microorganism of the genus *Shewanella,* more specifically, *Shewanella corallii, Shewanella oneidensis, Shewanella putrefaciens, Shewanella putrefaciens, Shewanella* sp. MR-4, *Shewanella* sp. BC20, *Shewanella xiamenensis, Shewanella decolorationis, Shewanella* sp. HN-41, *Shewanella baltica, Shewanella* sp. ISTPL2, and much more specifically, *Shewanella corallii* or *Shewanella oneidensis,* but is not limited thereto.

For still another example, the amino acid sequence of the LysE/ArgO family amino acid transporter of the present disclosure may be WP_115137742.1 derived from *Shewanella corallii* or WP_011072781.1 derived from *Shewanella oneidensis* MR-1, but is not limited thereto. It is obvious that proteins with LysE/ArgO family amino acid transporter activity, derived from various microorganisms of the genus *Shewanella,* are comprised.

As used herein, the term "microorganism of the genus *Shewanella"* is one of the marine bacteria that are able to survive in an environment both in the presence and absence of oxygen, and are known as bacteria having the function of inducing the reduction of toxic metals such as chromium and uranium and precipitating the same.

In the present disclosure, the LysE/ArgO family amino acid transporter protein derived from the strain of the genus *Shewanella* may have, comprise, or consist of an amino acid sequence represented by SEQ ID NO: 1 or SEQ ID NO: 3, or may essentially consist of the amino acid sequence.

In the present disclosure, the LysE/ArgO family amino acid transporter protein derived from the strain of the genus *Shewanella* may comprise the amino acid sequence of SEQ ID NO: 1 or SEQ ID NO: 3 or an amino acid sequence having at least 80%, 80.3%, 85%, 90%, 95%, 96%, 97%, 98%, 99%, 99.5%, 99.7% or 99.9% or more homology or identity thereto. Further, it is apparent that any protein having an amino acid sequence with deletion, modification, substitution, conservative substitution or addition in part of the sequence may also fall within the scope of the present disclosure, as long as the amino acid sequence has such a homology or identity and exhibits efficacy corresponding to that of the protein comprising the amino acid sequence of SEQ ID NO: 1 or SEQ ID NO: 3.

Examples thereof comprise those having sequence addition or deletion that do not alter the function of the protein of the present disclosure at the N-terminus, C-terminus of the amino acid sequence, and/or inside the amino acid sequence, a naturally occurring mutation, a silent mutation, or a conservative substitution.

The "conservative substitution" means the substitution of one amino acid with another amino acid having similar structural and/or chemical properties. Such an amino acid substitution may generally occur based on similarity in the polarity, charge, solubility, hydrophobicity, hydrophilicity, and/or amphipathic nature of residues. Usually, a conservative substitution may hardly affect or not affect the activity of the proteins or polypeptides.

As used herein, the term 'homology' or 'identity' means the degree of similarity between two given amino acid sequences or nucleotide sequences and may be expressed as a percentage. The terms 'homology and identity' may often be used interchangeably.

The sequence homology or identity of a conserved polynucleotide or polypeptide is determined by standard alignment algorithms, and the default gap penalty established by the used program may be used together. Substantially, homologous or identical sequences are generally capable of being hybridized with the entirety or a part of the sequence under moderately or highly stringent conditions. It is apparent that hybridization also comprises hybridization with a polynucleotide comprising a general codon or a codon considering codon degeneracy.

Whether any two polynucleotide or polypeptide sequences have homology, similarity, or identity may be determined using known computer algorithms such as the "FASTA" program, for example, using default parameters as in Pearson et al (1988)[Proc. Natl. Acad. Sci. USA 85]: 2444. Alternatively, the homology, similarity, or identity may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (comprising GCG program package (Devereux, J., et al, Nucleic Acids Research 12: 387 (1984)), BLASTP, BLASTN, FASTA (Atschul, [S.] [F.,] [ETAL, J MOLEC BIOL 215]: 403 (1990); Guide to Huge Computers, Martin J. Bishop, [ED.,] Academic Press, San Diego, 1994, and [CARILLO et al.](1988) SIAM J Applied Math 48: 1073). For example, BLAST of the National Center for Biotechnology Information, or ClustalW may be used to determine the homology, similarity, or identity.

The homology, similarity, or identity of polynucleotides or polypeptides may be determined by comparing sequence information using, for example, a GAP computer program such as Needleman et al. (1970), J Mol Biol. 48:443 as described in, for example, Smith and Waterman, Adv. Appl. Math (1981) 2:482. In summary, the GAP program may be defined as the value acquired by dividing the number of similarly aligned symbols (namely, nucleotides or amino acids) by the total number of symbols in the shorter of two sequences. The default parameters for the GAP program may comprise (1) a binary comparison matrix (comprising values of 1 for identity and 0 for non-identity) and a weighted comparison matrix of Gribskov et al(1986) Nucl. Acids Res. 14: 6745 (or EDNAFULL (EMBOSS version of NCBI NUC4.4) substitution matrix), as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

The gene encoding the LysE/ArgO family amino acid transporter protein of the present disclosure may be a gene named lysE and/or argO.

For example, the lysE gene and/or argO gene may be a polynucleotide encoding WP_115137742.1 derived from *Shewanella corallii* or WP_011072781.1 derived from Shewanella oneidensis MR-1, but is not necessarily limited thereto. It is obvious that the present disclosure comprises lysE gene and/or argO gene derived from various microorganisms of the genus *Shewanella,* which encode proteins having LysE/ArgO family amino acid transporter activity.

As used herein, the term "polynucleotide", which is a polymer of nucleotides, in which nucleotide monomers are linked in a long chain shape by covalent bonds, refers to a DNA or RNA strand having a predetermined or longer length, more specifically, a polynucleotide fragment encoding the protein.

The polynucleotide encoding the LysE/ArgO family amino acid transporter protein of the present disclosure may comprise a nucleotide sequence encoding the amino acid sequence represented by SEQ ID NO: 1 or SEQ ID NO: 3. In one embodiment of the present disclosure, the polynucleotide of the present disclosure may have or comprise a nucleotide sequence of SEQ ID NO: 2 or SEQ ID NO: 4. Further, the polynucleotide of the present disclosure may consist of or essentially consist of the nucleotide sequence of SEQ ID NO: 2 or SEQ ID NO: 4. Specifically, the LysE/ArgO family amino acid transporter protein may be encoded by the polynucleotide represented by the nucleotide sequence of SEQ ID NO: 2 or SEQ ID NO: 4.

In the polynucleotide of the present disclosure, various modifications may be made in the coding region as long as the amino acid sequence of the LysE/ArgO family amino acid transporter protein is not changed in consideration of codon degeneracy or codons preferred in organisms that are intended to express the LysE/ArgO family amino acid transporter protein of the present disclosure. Specifically, the polynucleotide of the present disclosure may have or comprise a nucleotide sequence having 70% or more, 75% or more, 76% or more, 85% or more, 90% or more, 95% or more, 96% or more, 97% or more, 98% or more homology or identity to the nucleotide sequence of SEQ ID NO: 2 or SEQ ID NO: 4, or may consist of or essentially consist of a nucleotide sequence having 70% or more, 75% or more, 76% or more, 85% or more, 90% or more, 95% or more, 96% or more, 97% or more, 98% or more homology or identity to the sequence of SEQ ID NO: 2 or SEQ ID NO: 4, but is not limited thereto.

Further, the polynucleotide of the present disclosure may comprise a probe that may be prepared from a known gene sequence, for example, any sequence without limitation as long as it is a sequence that is able to hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al.,Current Protocols in Molecular Biology, John Wiley & Sons, Inc., New York, 9.50-9.51, 11.7-11.8). Examples thereof comprise conditions under which polynucleotides having higher homology or identity, namely, polynucleotides having 70% or more, 75% or more, 76% or more, 85% or more, 90% or more, 95% or more, 96% or more, 97% or more, 98% or more, or 99% or more homology or identity are hybridized with each other while polynucleotides having lower homology or identity are not hybridized with each other, or conditions under which washing is performed once, specifically twice to three times at a salt concentration and temperature equivalent to 60°C, 1×SSC, 0.1% SDS, specifically 60°C, 0.1×SSC, 0.1% SDS, more specifically 68°C, 0.1×SSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments that are complementary to the entire sequence.

Specifically, a polynucleotide having homology or identity to the polynucleotide of the present disclosure may be detected using a hybridization condition comprising a hybridization step at a Tm value of 55°C and the above-described conditions. Further, the Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook et al., supra).

As used herein, the term "microorganism (or strain)" comprises all wild-type microorganisms or naturally or artificially genetically modified microorganisms, and it may be a microorganism in which a specific mechanism is weakened or enhanced due to an insertion of a foreign gene or an activity enhancement or weakening of an endogenous gene, and it may be a microorganism comprising a genetic modification for the production of a target polypeptide, protein, or product.

The strain of the present disclosure may be a microorganism naturally having the L-ornithine-producing ability or a microorganism prepared by providing the L-ornithine-producing ability to a strain having no L-ornithine-producing ability. For example, the strain may be a microorganism in which the activity of ornithine carbamoyltransferase subunit F (ArgF) and/or arginine repressor (ArgR) is weakened; and/or endogenous LysE protein is deleted. For another example, the strain may be a microorganism in which the LysE/ArgO family amino acid transporter protein derived from the strain of the genus *Shewanella* of the present disclosure or the polynucleotide encoding the same is introduced and thus L-ornithine export ability is improved, but is not limited thereto.

The strain of the present disclosure may be a microorganism in which the L-ornithine-producing ability is increased, as compared to a strain of the genus *Corynebacterium* without the LysE/ArgO family amino acid transporter protein derived from the strain of the genus *Shewanella* of the present disclosure or a wild-type strain of the genus *Corynebacterium.* Specifically, the strain of the present disclosure may be a recombinant microorganism introduced with the LysE/ArgO family amino acid transporter protein derived from *Shewanella corallii* or the LysE/ArgO family amino acid transporter protein derived from *Shewanella oneidensis* that has increased activity, as compared to the endogenous activity. The strain has an increased activity of the LysE/ArgO family amino acid transporter protein, as compared to the endogenous activity, and thus its L-ornithine export ability is improved, thereby having an improved L-ornithine-producing ability. In other words, the strain of the present disclosure may have an increased activity of the LysE/ArgO family amino acid transporter protein, as compared to the endogenous activity.

For example, the "unmodified microorganism in which the LysE/ArgO family amino acid transporter protein derived from the strain of the genus *Shewanella* is not introduced", which is a target strain for comparing whether or not the L-ornithine-producing ability increases, may be a *Corynebacterium glutamicum* strain having the L-ornithine-producing ability, in which serine at position 55 from the N-terminus of the amino acid sequence of endogenous ArgF is substituted with a stop codon, and glutamate at position 47 from the N-terminus of the amino acid sequence of endogenous ArgR is substituted with a stop codon; or a *Corynebacterium glutamicum* strain in which endogenous LysE is deleted, but is not limited thereto.

For example, the recombinant strain having the increased production ability may have an increased L-ornithine-producing ability of about 1% or more, specifically, about 1% or more, about 2% or more, about 5% or more, about 10% or more, about 15% or more, about 18% or more, about 18.9% or more, about 19% or more, about 19.1% or more, about 19.7% or more, about 20% or more, or about 20.3% or more (the upper limit is not particularly limited, but may be, for example, about 200% or less, about 150% or less, about 100% or less, about 50% or less, about 40% or less, about 30% or less, about 20% or less, or about 15% or less), as compared to that of the parent strain before modification or the unmodified microorganism, but the increased amount is not limited thereto as long as the production ability has an increased amount of a + value, as compared to the production ability of the parent strain before modification, the unmodified microorganism, or the microorganism in which the LysE/ArgO family amino acid transporter protein derived from the strain of the genus *Shewanella* or the polynucleotide encoding the same is not introduced, but is not limited thereto. In another example, the recombinant strain having the increased L-ornithine-producing ability may have an increased L-ornithine-producing ability of about 1.01 times or more, about 1.02 times or more, about 1.05 times or more, about 1.10 times or more, about 1.15 times or more, about 1.18 times or more, about 1.189 times or more, about 1.19 times or more, about 1.191 times or more, about 1.197 times or more, about 1.20 times or more, or about 1.203 times or more (the upper limit is not particularly limited, but may be, for example, about 10 times or less, about 5 times or less, about 3 times or less, or about 2 times or less), as compared to that of the parent strain before modification, the unmodified microorganism, or the microorganism in which the LysE/ArgO family amino acid transporter protein derived from the strain of the genus *Shewanella* or the polynucleotide encoding the same is not introduced, but is not limited thereto.

As used herein, the term "unmodified microorganism" does not exclude strains comprising mutations that may occur naturally in microorganisms, and may be a wild-type strain or a natural strain itself or may be a strain before the trait is changed by genetic variation due to natural or artificial factors. Further, the microorganism may refer to a strain in which the LysE/ArgO family amino acid transporter protein derived from the strain of the genus *Shewanella,* described herein, is not introduced or has not yet been introduced. **The** unmodified microorganism of the present disclosure does not exclude a strain comprising introduction or modification of another protein or another gene, in addition to the introduction of the LysE/ArgO family amino acid transporter protein derived from the strain of the genus *Shewanella,* or the polynucleotide encoding the same.

As used herein, the term "unmodified microorganism" may be used interchangeably with "strain before being modified", "microorganism before being modified", "unvaried strain", "unmodified strain", "unvaried microorganism", or "reference microorganism".

**The** microorganism of the present disclosure may be a microorganism in which the LysE/ArgO family amino acid transporter protein derived from the strain of the genus *Shewanella* or the polynucleotide encoding the same is introduced; or a microorganism (e.g., recombinant microorganism) which is genetically modified to introduce the LysE/ArgO family amino acid transporter protein derived from the strain of the genus *Shewanella* or the polynucleotide encoding the same, but is not limited thereto. The "endogenous activity" means the activity of a specific polypeptide originally possessed by a parent strain before the trait is changed, or a wild-type or unmodified microorganism, when the trait is changed by genetic variation due to natural or artificial factors. This may be used interchangeably with "activity before modification".

In another embodiment of the present disclosure, the microorganism of the present disclosure may be *Corynebacterium stationis, Corynebacterium glutamicum, Corynebacterium crudilactis, Corynebacterium deserti, Corynebacterium efficiens, Corynebacterium callunae, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* specifically, *Corynebacterium glutamicum.*

In the microorganism of the present disclosure, the activity of the LysE/ArgO family amino acid transporter may be enhanced by a known method, in addition to the introduction of the LysE/ArgO family amino acid transporter protein derived from the strain of the genus *Shewanella* of the present disclosure.

As used herein, the term "enhancement" of the polypeptide (e.g., protein specified by the name of each enzyme) activity means that the activity of the polypeptide is increased, as compared to the endogenous activity thereof. The enhancement may be used interchangeably with terms such as activation, up-regulation, overexpression, and increase, etc. In particular, the activation, enhancement, up-regulation, overexpression, and increase may comprise both cases in which an activity not originally possessed is exhibited, or the activity is enhanced, as compared to the endogenous activity or the activity before modification. The "endogenous activity" means the activity of a specific polypeptide originally possessed by a parent strain before the trait is changed or an unmodified microorganism, when the trait is changed by genetic variation due to natural or artificial factors. This may be used interchangeably with "activity before modification". The fact that the activity of a polypeptide is "enhanced", "up-regulated", "overexpressed", or "increased, as compared to the endogenous activity means that the activity of the polypeptide is improved as compared to the activity and/or concentration (expression level) of the specific polypeptide originally possessed by a parent strain before the trait is changed or an unmodified microorganism.

The enhancement may be achieved through the introduction of a foreign polypeptide or the enhancement of the activity of the endogenous polypeptide, and/or concentration (expression level). The enhancement of the activity of the polypeptide may be confirmed by an increase in the degree of activity and the expression level of the corresponding polypeptide or an increase in the amount of the product released from the corresponding polypeptide.

For the enhancement of the activity of the polypeptide, various methods well known in the art may be applied, and the method is not limited as long as the activity of the desired polypeptide may be enhanced as compared to that of the microorganism before modification. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the activity enhancement of the polypeptide of the present disclosure may be:
1) increase in the intracellular copy number of the polynucleotide encoding the polypeptide;
2) replacement of a gene expression regulatory region on a chromosome encoding the polypeptide with a sequence having stronger activity;
3) modification of a nucleotide sequence encoding an initiation codon or 5'-UTR region of the gene transcript encoding the polypeptide;
4) modification of the amino acid sequence of the polypeptide to enhance the activity of the polypeptide;
5) modification of the polynucleotide sequence encoding the polypeptide to enhance the activity of the polypeptide (e.g., modification of the polynucleotide sequence of the polypeptide gene to encode the polypeptide that has been modified to enhance the activity of the polypeptide);
6) introduction of a foreign polypeptide exhibiting the activity of the polypeptide or a foreign polynucleotide encoding the same;
7) codon optimization of the polynucleotide encoding the polypeptide;
8) analysis of the tertiary structure of the polypeptide to select and to modify or chemically modify the exposed site; or
9) a combination of two or more selected from 1) to 8), but is not particularly limited thereto.

More specifically,
1) the increase in the intracellular copy number of the polynucleotide encoding the polypeptide may be achieved by introducing, into a host cell, a vector which may replicate and function independently of the host and to which the polynucleotide encoding the corresponding polypeptide is operably linked. Alternatively, the increase may be achieved by introducing one copy or two or more copies of the polynucleotide encoding the corresponding polypeptide into a chromosome of a host cell. **The** introduction into the chromosome may be performed by introducing, into a host cell, a vector capable of inserting the polynucleotide into a chromosome of the host cell, but is not limited thereto. The vector is as described above.
2) The replacement of a gene expression regulatory region (or expression control sequence) on a chromosome encoding the polypeptide with a sequence exhibiting strong activity may be, for example, the occurrence of variation in a sequence due to deletion, insertion, nonconservative or conservative substitution, or a combination thereof, or replacement with a sequence exhibiting stronger activity so that the activity of the expression regulatory region is further enhanced. The expression regulatory region may comprise, but is not particularly limited to, a promoter, an operator sequence, a sequence encoding a ribosome binding site, a sequence controlling the termination of transcription and translation, etc. For example, the replacement may be to replace the original promoter with a strong promoter, but is not limited thereto.

Examples of known strong promoters comprise CJ1 to CJ7 promoters (US 7662943 B2), a lac promoter, a trp promoter, a trc promoter, a tac promoter, a lambda phage PR promoter, a PL promoter, a tet promoter, a gapA promoter, a SPL7 promoter, a SPL13(sm3) promoter (US 10584338 B2), an O2 promoter (US 10273491 B2), a tkt promoter, an yccA promoter, etc., but is not limited thereto.
3) The modification of a nucleotide sequence encoding an initiation codon or 5'-UTR region of the gene transcript encoding the polypeptide may be, for example, substitution with a nucleotide sequence encoding another initiation codon having a higher polypeptide expression rate as compared to an endogenous initiation codon, but is not limited thereto.
4) and 5) The modification of the amino acid sequence or the polynucleotide sequence may involve the occurrence of variation in the sequence due to deletion, insertion, nonconservative or conservative substitution of the amino acid sequence of the polypeptide or the polynucleotide sequence encoding the polypeptide, or a combination thereof, or replacement with an amino acid sequence or polynucleotide sequence improved to exhibit stronger activity or an amino acid sequence or polynucleotide sequence improved to be more active so that the activity of the polypeptide is enhanced, but is not limited thereto. The replacement may be specifically performed by inserting a polynucleotide into a chromosome by homologous recombination, but is not limited thereto. The vector used here may further comprise a selection marker for the confirmation of chromosome insertion.
6) The introduction of a foreign polynucleotide exhibiting the activity of the polypeptide may involve the introduction of a foreign polynucleotide into a host cell, the foreign polynucleotide encoding a polypeptide exhibiting activity identical or similar to that of the polypeptide. There is no limitation on its origin or sequence as long as the foreign polynucleotide exhibits activity identical or similar to that of the polypeptide. The method used in the introduction may be performed by appropriately selecting a known transformation method by those skilled in the art. As the introduced polynucleotide is expressed in the host cell, the polypeptide may be produced and the activity thereof may be increased.
7) The codon optimization of the polynucleotide encoding the polypeptide may involve codon optimization of an endogenous polynucleotide so as to enhance transcription or translation in a host cell, or codon optimization of a foreign polynucleotide so as to achieve optimized transcription and translation in a host cell.
8) The analysis of the tertiary structure of the polypeptide to select and to modify or chemically modify the exposed site may involve, for example, determining a template protein candidate according to the degree of similarity of the sequence by comparing the sequence information of a polypeptide to be analyzed with a database storing the sequence information of known proteins, confirming the structure based on this, and modifying or chemically modifying the exposed site to be modified or chemically modified.

Such enhancement of the polypeptide activity may result in an increase in the activity or concentration/expression level of the corresponding polypeptide, based on the activity or concentration of the polypeptide expressed in a wild-type or a microbial strain before being modified, or an increase in the amount of a product produced from the corresponding polypeptide, but is not limited thereto.

The modification of a part or the entirety of the polynucleotide in the microorganism of the present disclosure may be induced by: (a) homologous recombination using a vector for chromosomal insertion in a microorganism or genome editing using engineered nuclease (e.g., CRISPR-Cas9), and/or (b) treatment with light, such as ultraviolet light and radiation, and/or chemicals, but is not limited thereto. The method of modifying a part or the entirety of the gene may comprise a method using DNA recombinant technology. For example, a nucleotide sequence or vector comprising a nucleotide sequence homologous to a target gene may be introduced into the microorganism to bring about homologous recombination, resulting in the deletion of a part or the entirety of the gene. The nucleotide sequence or vector to be introduced may comprise a dominant selection marker, but is not limited thereto.

The vector of the present disclosure may comprise a DNA construct comprising a nucleotide sequence of a polynucleotide encoding a desired polypeptide which is operably linked to a suitable expression regulatory region (or expression control sequence) so that the desired polypeptide may be expressed in a suitable host. The expression regulatory region may comprise a promoter capable of initiating transcription, any operator sequence for controlling the transcription, a sequence encoding a suitable mRNA ribosome binding site, and a sequence controlling termination of transcription and translation. The vector may be transformed into a suitable host cell, and then replicate or function independently of the host genome, or may be integrated into the genome itself.

The vector used in the present disclosure is not particularly limited, but any vector known in the art may be used. Examples of commonly used vectors comprise natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pBR system, pUC system, pBluescript II system, pGEM system, pTZ system, pCL system, pET system, etc. may be used as a plasmid vector. Specifically, pDZ, pDC, pDCM2, pACYC177, pACYC184, pCL, pECCG117, pUC19, pBR322, pMW118, pCC1BAC vector, etc. may be used.

For example, a polynucleotide encoding a desired polypeptide may be inserted into a chromosome through a vector for intracellular chromosome insertion. Insertion of the polynucleotide into the chromosome may be performed by any method known in the art, for example, homologous recombination, but is not limited thereto. The vector may further comprise a selection marker for the confirmation of chromosome insertion. The selection marker is for selecting the cells transformed with vectors, i.e., for confirming the insertion of a desired nucleic acid molecule, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector comprising a polynucleotide encoding a target polypeptide is introduced into a host cell or a microorganism so that the polypeptide encoded by the polynucleotide may be expressed in the host cell. The transformed polynucleotide may be located regardless of the position, either by being inserted into the chromosome of the host cell or located outside the chromosome as long as it may be expressed in the host cell. Further, the polynucleotide comprises DNA and/or RNA encoding a desired polypeptide. The polynucleotide may be introduced in any form as long as it may be introduced into a host cell and expressed. For example, the polynucleotide may be introduced into a host cell in the form of an expression cassette, which is a gene construct comprising all elements required for autonomous expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, the term "operably linked" means that the polynucleotide sequence is functionally linked to a promoter sequence that initiates and mediates transcription of the polynucleotide encoding the desired polypeptide of the present disclosure.

In still another embodiment of the present disclosure, the microorganism of the present disclosure may be a microorganism in which the L-ornithine-producing ability is enhanced by additionally enhancing the activity of a part of the proteins in the L-ornithine biosynthetic pathway or by additionally weakening the activity of a part of the proteins in the L-ornithine degradation pathway.

Specifically, the microorganism of the present disclosure may be a microorganism in which the activity of endogenous ArgF is additionally weakened, or the argF gene encoding the same is additionally deleted, or a microorganism in which the activity of endogenous ArgR is additionally weakened, or the argR gene encoding the same is additionally deleted, and/or a microorganism in which the activity of LysE is additionally weakened, or the lysE gene encoding the same is additionally deleted.

An amino acid sequence of the ArgF, ArgR, or LysE may be obtained from NCBI's Genbank which is a known database, etc. For example, the amino acid sequence of ArgF of the present disclosure may comprise ANU33618.1 (SEQ ID NO: 5) derived from *Corynebacterium glutamicum* ATCC 13869 or an amino acid sequence having 80% or more sequence identity thereto, the amino acid sequence of ArgR of the present disclosure may comprise ANU33619.1 (SEQ ID NO: 7) derived from *Corynebacterium glutamicum*ATCC 13869 or an amino acid sequence having 80% or more sequence identity thereto, and the amino acid sequence of LysE may comprise ANU33473.1 (SEQ ID NO: 9) derived from *Corynebacterium glutamicum* ATCC 13869 or an amino acid sequence having 80% or more sequence identity thereto, but is not limited thereto, and it is obvious that proteins with ArgF, ArgR, or LysE activity, derived from various origins, are comprised.

However, the weakening of the ArgF, ArgR, and/or LysE protein activity or the deletion of the argF, argR, and/or lysE gene is only one example and is not limited thereto, and the microorganism of the present disclosure may be a microorganism in which the activities of the proteins in various known L-ornithine biosynthetic pathways are enhanced or the activities of the proteins in the degradation pathway are weakened.

As used herein, the term "weakening" of the activity of the polypeptide has a concept encompassing all of the decrease of activity or the absence of activity, as compared to the endogenous activity. The weakening may be used interchangeably with terms such as inactivation, deficiency, down-regulation, decrease, reduction, attenuation, etc.

The weakening may also comprise a case where the activity of the polypeptide itself is decreased or eliminated due to variation of the polynucleotide encoding the polypeptide, etc., as compared to the activity of the polypeptide originally possessed by the microorganism, a case where the overall polypeptide activity level and/or concentration (expression level) in the cell is low due to inhibition of the expression of the gene of the polynucleotide encoding the polypeptide or due to inhibition of translation into the polypeptide, as compared to that of the natural strain, a case where the polynucleotide is not expressed at all, and/or a case where the polypeptide activity is absent even when the polynucleotide is expressed. The "inactivation, deficiency, decrease, down-regulation, reduction, or attenuation" of the activity of a polypeptide as compared to the endogenous activity thereof means that the activity of the polypeptide is lowered as compared to the activity of a specific polypeptide originally possessed by a parent strain before the trait is changed or an unmodified microorganism.

Such weakening of the activity of the polypeptide may be performed by any method known in the art, but is not limited thereto, and may be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the activity of the polypeptide of the present disclosure may be achieved by:
1) deletion of the entirety or a part of the gene encoding the polypeptide;
2) modification of an expression control region (or expression control sequence) to reduce the expression of a gene encoding the polypeptide;
3) modification of the amino acid sequence constituting the polypeptide to eliminate or weaken the activity of the polypeptide (e.g., deletion/substitution/addition of one or more amino acids in the amino acid sequence);
4) modification of the gene sequence encoding the polypeptide to eliminate or weaken the activity of the polypeptide (e.g., deletion/substitution/addition of one or more nucleotides in the nucleotide sequence of the polypeptide gene to encode the polypeptide which is modified to eliminate or weaken the activity of the polypeptide);
5) modification of a nucleotide sequence encoding an initiation codon or 5'-UTR region of the gene transcript encoding the polypeptide;
6) introduction of an antisense oligonucleotide (e.g., antisense RNA) complementarily binding to the gene transcript encoding the polypeptide;
7) addition of a sequence complementary to the Shine-Dalgarno sequence of the gene encoding the polypeptide to the upstream of the Shine-Dalgarno sequence in order to form a secondary structure that makes the attachment of ribosomes impossible;
8) addition of a promoter, which is to be reverse-transcribed, to the 3' end of the open reading frame (ORF) of the gene sequence encoding the polypeptide (reverse transcription engineering, RTE); or
9) combination of two or more selected from 1) to 8), but is not particularly limited thereto.

For example,
1) The deletion of a part or the entirety of the gene encoding the polypeptide may involve the elimination of the entirety of the polynucleotide encoding an endogenous target polypeptide in the chromosome, replacement with a polynucleotide with the deletion of some nucleotides, or replacement with a marker gene.
2) The modification of an expression control region (or expression control sequence) may involve the mutation of the expression control region (or expression control sequence) through deletion, insertion, non-conservative or conservative substitution, or a combination thereof, or the replacement with a sequence having weaker activity. The expression control region comprises a promoter, an operator sequence, a sequence for encoding a ribosomal binding site, and a sequence for controlling the termination of transcription and translation, but is not limited thereto.
3) and 4) The modification of the amino acid sequence or the polynucleotide sequence may involve the mutation of the sequence through deletion, insertion, non-conservative or conservative substitution, or a combination thereof in the amino acid sequence of the polypeptide or the polynucleotide sequence encoding the polypeptide, or a replacement with an amino acid sequence or polynucleotide sequence improved to have weaker activity, or an amino acid sequence or polynucleotide sequence improved to have no activity, so as to weaken the activity of the polypeptide, but is not limited thereto. For example, the expression of the gene may be inhibited or weakened by introducing a mutation into the polynucleotide sequence to form a stop codon, but is not limited thereto.
5) The modification of a nucleotide sequence encoding an initiation codon or 5'-UTR region of the gene transcript encoding the polypeptide may involve, for example, the substitution with a nucleotide sequence encoding, rather than an endogenous initiation codon, another initiation codon having a lower expression rate of the polypeptide, but is not limited thereto.
6) The introduction of an antisense oligonucleotide (e.g., antisense RNA) complementarily binding to the gene transcript encoding the polypeptide may refer to, for example, the literature [Weintraub, H. et al., Antisense-RNA as a molecular tool for genetic analysis, Reviews - Trends in Genetics, Vol. 1(1) 1986].
7) The addition of a sequence complementary to the Shine-Dalgarno sequence of the gene encoding the polypeptide to the upstream of the Shine-Dalgarno sequence so as to form a secondary structure that makes the attachment of ribosomes impossible may involve making mRNA translation impossible or reducing the rate thereof.

Further, 8) the addition of a promoter, which is to be reverse-transcribed, to the 3' end of the open reading frame (ORF) of the gene sequence encoding the polypeptide (reverse transcription engineering, RTE) may involve making an antisense nucleotide complementary to the gene transcript encoding the polypeptide to weaken the activity.

Still another aspect of the present disclosure provides a method of producing L-ornithine, the method comprising the step of culturing, in a medium, a recombinant microorganism of the genus *Corynebacterium* having L-ornithine-producing ability, the recombinant microorganism comprising the LysE/ArgO family amino acid transporter protein derived from a strain of the genus *Shewanella* or the polynucleotide encoding the LysE/ArgO family amino acid transporter protein.

The method of producing L-ornithine of the present disclosure may comprise the step of culturing, in a medium, a microorganism which is genetically modified to comprise the LysE/ArgO family amino acid transporter protein derived from a strain of the genus *Shewanella* of the present disclosure or the polynucleotide encoding the same.

As used herein, the term "culturing" refers to growing the microorganism of the present disclosure in appropriately adjusted environmental conditions. The culture procedure of the present disclosure may be performed according to appropriate media or culture conditions known in the art. Such a culture procedure may be easily adjusted according to the selected microorganism by a person skilled in the art. Specifically, the culturing may be in a batch type, a continuous type, and/or a fed-batch type, but is not limited thereto.

As used herein, the "medium" refers to a mixture comprising, as main ingredients, nutrient materials required for culturing the microorganism of the present disclosure, wherein the medium supplies nutrient materials comprising water essential for survival and growth, growth factors, etc. Specifically, as for the media and other culture conditions used for culturing the microorganism of the present disclosure, any medium that is used for the usual culture of microorganisms may be used without particular limitation. However, the microorganism of the present disclosure may be cultured under aerobic conditions in a common medium comprising appropriate carbon sources, nitrogen sources, phosphorus sources, inorganic compounds, amino acids, and/or vitamins, etc., while controlling the temperature, pH, etc.

Specifically, the culture medium for the microorganism of the genus *Corynebacterium* may be found in the literature ["Manual of Methods for General Bacteriology" by the American Society for Bacteriology (Washington D.C., USA, 1981)].

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fish or decomposition products thereof, defatted soybean cake or degradation products thereof, etc. may be used. These nitrogen sources may be used alone or in a combination of two or more thereof, but are not limited thereto.

The phosphorus sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These constituent ingredients or precursors may be added to the medium in a batch or continuous manner. However, the present disclosure is not limited thereto.

Further, the pH of the medium may be adjusted by adding compounds, such as ammonium hydroxide, potassium hydroxide, ammonia, phosphoric acid, and sulfuric acid, to the medium in an appropriate manner during the culture of the microorganism of the present disclosure. In addition, an anti-foaming agent, such as a fatty acid polyglycol ester, may be used to suppress foam formation during the culture. In addition, oxygen or oxygen-containing gas may be injected into the medium to maintain the aerobic state of the medium, or no gas may be injected, or nitrogen, hydrogen, or carbon dioxide gas may be injected to maintain the anaerobic or non-aerobic state of the medium, but is not limited thereto.

In the culture of the present disclosure, the culture temperature may be maintained at 20°C to 45°C, specifically, at 25°C to 40°C, and the culture may be performed for about 10 hours to about 160 hours, but is not limited thereto.

The L-ornithine produced by the culturing of the present disclosure may be released into the medium or may remain in cells.

The method of producing L-ornithine of the present disclosure may further comprise the steps of preparing the microorganism of the present disclosure, preparing a medium for culturing the microorganism, or a combination thereof (regardless of the order, in any order), for example, before the culturing step.

The method of producing L-ornithine of the present disclosure may further comprise the step of recovering L-ornithine from a medium resulting from the culturing (a medium in which culturing has been performed) or the cultured microorganism. The recovering step may be further comprised after the culturing step.

The recovering may involve collecting the desired L-ornithine by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, and affinity chromatography, etc., HPLC, and a combination of these methods may be used, and the desired L-ornithine may be recovered from the medium or microorganism by using an appropriate method known in the art.

In addition, the method of producing L-ornithine of the present disclosure may further comprise a purification step. The purification may be performed by using an appropriate method known in the art. In an exemplary embodiment, when the method of producing L-ornithine of the present disclosure comprises both the recovering step and the purification step, the recovering step and the purification step may be performed continuously or discontinuously regardless of the order, or may be performed simultaneously or integrated into one step, but is not limited thereto.

The microorganism of the present disclosure may be a microorganism in which the L-ornithine-producing ability is enhanced by additionally enhancing the activity of a part of the proteins in the L-ornithine biosynthetic pathway or by additionally weakening the activity of a part of the proteins in the L-ornithine degradation pathway.

For example, the microorganism of the present disclosure may be a microorganism in which the activity of endogenous ArgF is additionally weakened, or the argF gene encoding the same is additionally deleted, or a microorganism in which the activity of endogenous ArgR is additionally weakened, or the argR gene encoding the same is additionally deleted, and/or a microorganism in which the activity of LysE is additionally weakened, or the lysE gene encoding the same is additionally deleted.

In the method of the present disclosure, the strain of the genus *Shewanella,* the LysE/ArgO family amino acid transporter protein, the polynucleotide, the vector, and the microorganism are as described in other aspects.

Still another aspect of the present disclosure provides a composition for producing L-ornithine, the composition comprising the recombinant microorganism of the genus *Corynebacterium* comprising the LysE/ArgO family amino acid transporter protein derived from the strain of the genus *Shewanella* or the polynucleotide encoding the LysE/ArgO family amino acid transporter protein; a culture in which the microorganism is cultured; or a combination thereof.

The composition of the present disclosure may further comprise any appropriate excipient that is usually used in the composition for producing L-ornithine, and such excipients may comprise, for example, a preserving agent, a wetting agent, a dispersing agent, a suspending agent, a buffering agent, a stabilizing agent, an isotonic agent, etc., but are not limited thereto.

Still another aspect of the present disclosure provides a method of preparing the recombinant microorganism of the genus *Corynebacterium* for producing L-ornithine, the method comprising the step of introducing the LysE/ArgO family amino acid transporter protein derived from the strain of the genus *Shewanella* or the polynucleotide encoding the LysE/ArgO family amino acid transporter protein.

Still another aspect of the present disclosure provides use of the recombinant microorganism of the genus *Corynebacterium* in producing L-ornithine, in which the recombinant microorganism is introduced with the LysE/ArgO family amino acid transporter protein derived from the strain of the genus *Shewanella* or the polynucleotide encoding the LysE/ArgO family amino acid transporter protein.

The strain of the genus *Shewanella,* the LysE/ArgO family amino acid transporter protein, the introduction, the microorganism of the genus *Corynebacterium,* etc. are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1: Selection of proteins having L-ornithine export activity and Construction of plasmid

LysE/ArgO family amino acid transporter (WP_115137742.1) (SEQ ID NO: 1) which is a membrane protein derived from *Shewanella corallii* A687 and LysE/ArgO family amino acid transporter (WP_011072781.1) (SEQ ID NO: 3)which is a membrane protein derived from *Shewanella oneidensis* MR-1 were selected as proteins having high L-ornithine export activity, and to amplify nucleotide sequences of the genes encoding the same, information (NC_004347.2) about the genes encoding the membrane proteins and their surrounding nucleotide sequences was obtained from NIH GenBank. Based on the corresponding sequence information, DNA synthesis was performed (Cosmo genetech, Korea).

Vectors for introducing the selected membrane proteins, each derived from *Shewanella corallii* A687 and *Shewanella oneidensis* MR-1, into an L-ornithine-producing strain were constructed, respectively. First, ANU34435.1, which is one of the transposases existing in the genome of wild-type *Corynebacterium glutamicum* ATCC 13869 (NZ_CP016335.1), was used as an insertion site. The genome of wild-type *Corynebacterium glutamicum* ATCC 13869 was used as a template, and homologous recombinant A arm was amplified using a primer pair of SEQ ID NOS: 11 and 12, and homologous recombinant B arm was amplified using a primer pair of SEQ ID NOS: 13 and 14.

To obtain a gene fragment (SEQ ID NO: 2) encoding the membrane protein derived from *Shewanella corallii* A687, the synthesized DNA was used as a template and a primer pair of SEQ ID NOS: 15 and 16 was used, and to obtain the gapA promoter, the genomic DNA (NC_006958.1) of wild-type *Corynebacterium glutamicum* ATCC 13032 was used as a template and a primer pair of SEQ ID NOS: 19 and 20 was used to perform PCR at 95°C for 2 minutes, followed by 25 cycles of denaturation at 95°C for 1 minute, annealing at 55°C for 1 minute, and polymerization at 72°C for 1 minute, and then at 72°C for 5 minutes. At this time, Solg^{™} Pfu-X DNA polymerase was used for PCR.

To obtain a gene fragment (SEQ ID NO: 4) encoding the membrane protein derived from *Shewanella oneidensis* MR-1, the synthesized DNA was used as a template and a primer pair of SEQ ID NOS: 17 and 18 was used to perform PCR under the same conditions as above.

The amplified gapA promoter region, the gene fragment encoding the membrane protein derived from *Shewanella corallii* A687, or the gene fragment encoding the membrane protein derived from *Shewanella oneidensis* MR-1, the homologous recombinant arm gene fragment, and a vector pDCM2 (WO2021-187781 A1) digested with Sall and BamHI restriction enzymes were ligated using the Gibson assembly (DG Gibson et al., NATURE METHODS, VOL.6 NO.5, MAY 2009, NEBuilder HiFi DNA Assembly Master Mix) method, transformed into *E*. *coli* DH5α, and plated on an LB solid medium containing kanamycin (25 mg/l). To select colonies transformed with the vector comprising the gene encoding the membrane protein derived from *Shewanella corallii* A687 or the membrane protein derived from *Shewanella oneidensis* MR-1, PCR was performed using a primer pair of SEQ ID NOS: 21 and 22. Plasmids were extracted from the selected colonies using a plasmid prep kit (QIAGEN), and these plasmids were named pDCM2-PgapA-Sco and pDCM2-PgapA-Son, respectively.

Sequences of the primers used here are as in Table 1 below.

**[Table 1]**

| SEQ ID NO. | Sequence name | Sequence |
|---|---|---|
| 11 | primer 1 | CGGTACCCGGGGATCCTTTTAGATTCC CAGTGCAG |
| 12 | primer 2 | CGCGTGGGCTTTGGGCTGAG |
| 13 | primer 3 | TGCATTGGTGGAGCTAGCTG |
| 14 | primer 4 | ATGCCTGCAGGTCGACGCTATGCAGTG GAGATGTC |
| 15 | primer 5_ Sco | CTTTAGAGGAGACACAACATGCAGACA GCATTTATTC |
| 16 | primer 6_ Sco | TAGCTCCACCAATGCATCAACCTTTAAT ATATGGC |
| 17 | primer 5_Son | CTTTAGAGGAGACACAACATGCAAACG GCGTTTATTC |
| 18 | primer 6_Son | TAGCTCCACCAATGCATTAAATTGCGGC AAGGTAT |
| 19 | primer 7 | GCCCAAAGCCCACGCGAACGACCGAG CCTATTGGG |
| 20 | primer 8 | GTTGTGTCTCCTCTAAAGATTG |
| 21 | primer 9 | TATTACGCCAGCTGGCGAAA |
| 22 | primer 10 | GCTTTACACTTTATGCTTCC |

### Example 2: Preparation of L-ornithine-producing strain and lysE-deleted strain

In order to prepare an L-ornithine producing strain, a vector was constructed, which substitutes a stop codon for serine at position 55 from the N-terminus of the amino acid sequence of ArgF (ANU33618.1) (SEQ ID NO: 5) derived from wild-type *Corynebacterium glutamicum.* The genome of wild-type *Corynebacterium glutamicum* ATCC 13869 was used as a template, and homologous recombinant A arm was amplified using a primer pair of SEQ ID NOS: 23 and 24, and homologous recombinant B arm was amplified using a primer pair of SEQ ID NOS: 25 and 26. Thereafter, a plasmid was obtained in the same manner as in Example 1, and this plasmid was named pDCM2-argF (S55*).

To prepare a strain with further improved L-ornithine-producing ability, a vector was constructed, which substitutes a stop codon for glutamate at position 47 from the N-terminus of the amino acid sequence of ArgR (ANU33619.1) (SEQ ID NO: 7) derived from wild-type *Corynebacterium glutamicum.* The genome of wild-type *Corynebacterium glutamicum* ATCC 13869 was used as a template, and homologous recombinant A arm was amplified using a primer pair of SEQ ID NOS: 27 and 28, and homologous recombinant B arm was amplified using a primer pair of SEQ ID NOS: 29 and 30. Thereafter, a plasmid was obtained by the method described above, and this plasmid was named pDCM2-argR(E47*).

Wild-type *C. glutamicum* ATCC 13869 was transformed with a pDCM2-argF(S55*) vector by electroporation (Appl. Microbiol.Biotechnol. (1999) 52:541-545), and then a second crossover process was performed to obtain a strain, in which serine at position 55 from the N-terminus of the amino acid sequence of ArgF was substituted with a stop codon. PCR and sequencing analysis were performed using a primer pair of SEQ ID NOS: 23 and 26 which are capable of amplifying the adjacent region comprising the position where the corresponding gene was inserted, and the corresponding genetic manipulation was confirmed. The strain thus obtained was named C. gl::argF*.

C. gl::argF* was transformed with the pDCM2-argR(E47*) vector, and in the same manner as above, a strain was obtained, in which glutamate at position 47 from the N-terminus of the amino acid sequence of ArgR was substituted with a stop codon. PCR and sequencing analysis were performed using a primer pair of SEQ ID NOS: 27 and 30 which are capable of amplifying the adjacent region comprising the position where the corresponding gene was inserted, and the corresponding genetic manipulation was confirmed. The strain thus obtained was named C. gl::argF*_argR*.

Next, to construct a vector for deleting the open reading frame (ORF) of wild-type *Corynebacterium glutamicum* LysE (ANU33473.1) (SEQ ID NO: 9) to prepare a LysE-deleted strain, the genome of wild-type *Corynebacterium glutamicum* ATCC13869 was used as a template, and homologous recombinant A arm was amplified using a primer pair of SEQ ID NOS: 31 and 32, and homologous recombinant B arm was amplified using a primer pair of SEQ ID NOS: 33 and 34. The homologous recombinant arm gene fragments were cloned into a vector pDCM2 digested with Sall and BamHI restriction enzymes in the same manner as in Example 1, and transformed into *E*. *coli* DH5α. To select the transformed colonies, PCR was performed using a primer pair of SEQ ID NOS: 21 and 22. A plasmid was extracted from the selected colonies using a plasmid prep kit (QIAGEN), and this plasmid was named pDCM2-△lysE.

The pDCM2-△lysE plasmid was transformed into the L-ornithine-producing strain C. gl::argF*_argR* by electroporation, and then a second crossover process was performed to obtain a strain, in which the gene (lysE) encoding LysE was deleted. PCR and sequencing analysis were performed using a primer pair of SEQ ID NOS: 31 and 34 which are capable of amplifying the adjacent region comprising the position where the corresponding gene was inserted, and the corresponding genetic manipulation was confirmed. The strain thus obtained was named C. gl::argF*_argR*_△lysE.

Sequences of the primers used here are as in Table 2 below.

**[Table 2]**

| SEQ ID NO. | Sequence name | Sequence |
|---|---|---|
| 23 | primer 11 | CGGTACCCGGGGATCCTGACCCCAGGCA AGCACGG |
| 24 | primer 12 | GAAGCGAGTACGAGTTTAAGTCTTATC |
| 25 | primer 13 | AAACTCGTACTCGCTTCTCC |
| 26 | primer 14 | ATGCCTGCAGGTCGACCGGCGCCGGCA ACCTCGTC |
| 27 | primer 15 | CGGTACCCGGGGATCCCTCGTGCGGAAT TCGTGGAG |
| 28 | primer 16 | ATCCAGCAGCAATTCAGACA |
| 29 | primer 17 | CTGAATTGCTGCTGGATTAAGGCATCGAT ATCACCCA |
| 30 | primer 18 | ATGCCTGCAGGTCGACCCTTCATTTTAAG TTCCTTG |
| 31 | primer 19 | CGGTACCCGGGGATCCATTGCCTCACCA AAACCTTC |
| 32 | primer 20 | CGTGACCTATGGAAGTACTTAAG |
| 33 | primer 21 | GTACTTCCATAGGTCACGTTAGTTTTCGC GGGTTTTAG |
| 34 | primer 22 | ATGCCTGCAGGTCGACGTTTTGGTGGTT CCCGAAAG |

### Example 3: Preparation of strains introduced with gene of exogenous LysE/ArgO family amino acid transporter protein

To prepare strains in which the exogenous membrane protein gene was introduced, the pDCM2-PgapA-Sco and the pDCM2-PgapA-Son vectors constructed in Example 1 were transformed into the L-ornithine-producing strain C. gl::argF*_argR* and the lysE-deleted strain C. gl::argF*_argR*_△lysE, which were prepared in Example 2, by electroporation, respectively and then a second crossover process was performed to obtain a strain, in which PgapA-Sco was inserted, and a strain, in which PgapA-Son was inserted. PCR and sequencing analysis were performed using a primer pair of SEQ ID NOS: 26 and 29 which are capable of amplifying the adjacent region comprising the position where the corresponding gene was inserted, and the corresponding genetic manipulation was confirmed. The strains thus obtained were named C. gl::argF*_argR*_PgapA-Sco, C. gl::argF*_argR*_△lysE_PgapA-Sco, C. gl::argF*_argR*_PgapA-Son, and C. gl::argF*_argR*_△lysE_PgapA-Son, respectively.

### Example 4: Comparison of L-ornithine-producing ability between strains introduced with exogenous LysE/ArgO family amino acid transporter protein

Wild-type *Corynebacterium glutamicum* ATCC 13869 and the strains prepared in Example 3 and the strains prepared in Example 2 were cultured by the following method to compare the cell mass, sugar consumption, and L-ornithine-producing ability.

First, each strain was inoculated into a 250 ml corner-baffled flask comprising 25 ml of a seed medium, and cultured at 30°C for 20 hours with shaking at 200 rpm. 1 ml of the seed culture was inoculated into a 250 ml corner-baffled flask comprising 24 ml of a production medium, and cultured at 33°C for 42 hours with shaking at 200 rpm. After the culture was completed, the production of L-ornithine was measured using HPLC, and the results are shown in Tables 3 and 4 below.

### <Seed medium (pH 7.0)>

20 g of glucose, 10 g of peptone, 5 g of yeast extract, 1.5 g of urea, 4 g of KH₂PO₄, 8 g of K₂HPO₄, 0.5 g of MgSO₄·7H₂O, 0.1 mg of biotin, 1 mg of thiamine HCl, 22 mg of calcium pantothenate, 2 mg of nicotinamide (based on 1 liter of distilled water)

### <Production medium (pH 7.0)>

50 g of raw sugar, 25 g of (NH₄)₂SO₄, 1 g of yeast extract, 0.55 g of KH₂PO₄, 0.6 g of MgSO₄·7H₂O, 0.2 g of L-arginine, 0.9 mg of biotin, 4.5 mg of thiamine HCl, 4.5 mg of calcium pantothenate, 30 mg of nicotinamide, 9 mg of MnSO₄, 9 mg of FeSO₄, 0.45 mg of ZnSO₄, 0.45 mg of CuSO₄, 30 g of CaCO₃ (based on 1 liter of distilled water)

**[Table 3]**

| Strain | OD₅₆₂ | Sugar consumptio n (g/L) | L-ornithine production (g/L) | L-ornithine yield (%) |
|---|---|---|---|---|
| *C. glutamicum* WT | 95.0 | 50.0 | 0.0 | 0.0 |
| C. gl::argF*_argR* | 37.7 | 50.0 | 17.8 | 35.6 |
| C. gl::argF*_argR*_△lysE | 66.6 | 50.0 | 1.2 | 2.4 |
| **C. gl::argF*_argR*_PgapA-Sco** | **36.3** | **50.0** | **19.7** | **39.4** |
| **C. gl::argF*_argR*_△lysE_PgapA-Sco** | **38.1** | **50.0** | **18.9** | **37.8** |

**[Table 4]**

| Strain | OD₅₆₂ | Sugar consumption (g/L) | L-ornithine production (g/L) | L-ornithine yield (%) |
|---|---|---|---|---|
| *C. glutamicum* WT | 96.2 | 50.0 | 0.0 | 0.0 |
| C. gl::argF*_argR* | 37.6 | 50.0 | 17.9 | 35.8 |
| C. gl::argF*_argR*_△lysE | 66.5 | 50.0 | 1.3 | 2.6 |
| **C. gl::argF*_argR*_PgapA-Son** | **37.5** | **50.0** | **20.3** | **40.6** |
| **C. gl::argF*_argR*_△lysE_PgapA-Son** | **38.7** | **50.0** | **19.1** | **38.2** |

As shown in Table 3, the C. gl::argF*_argR*_△lysE_PgapA-Sco strain, in which the gene encoding the LysE/ArgO family amino acid transporter protein which is a membrane protein derived from *Shewanella corallii* A687 was introduced as the exogenous membrane protein into the lysE-deleted strain, showed remarkably increased L-ornithine-producing ability and L-ornithine yield, as compared to the lysE-deleted, C. gl::argF*_argR*_△lysE strain. Further, the C. gl::argF*_argR*_PgapA-Sco strain, in which the membrane protein derived from *Shewanella corallii* A687 was introduced, showed increased L-ornithine-producing ability and L-ornithine yield, as compared to C. gl::argF*_argR*.

As shown in Table 4, the C. gl::argF*_argR*_△lysE_PgapA-Son strain, in which the gene encoding the LysE/ArgO family amino acid transporter protein which is a membrane protein derived from *Shewanella oneidensis* MR-1 was introduced as the exogenous membrane protein into the lysE-deleted strain, showed remarkably increased L-ornithine-producing ability and L-ornithine yield, as compared to the lysE-deleted, C. gl::argF*_argR*_△lysE strain. Further, the C. gl::argF*_argR*_PgapA-Son strain, in which the membrane protein derived from *Shewanella oneidensis* MR-1 was introduced, showed increased L-ornithine-producing ability and L-ornithine yield, as compared to C. gl::argF*_argR*.

Based on the above description, a person skilled in the art to which the present disclosure pertains can understand that the present disclosure may be embodied in other specific forms without departing from the technical spirit or essential characteristics thereof. In this regard, the embodiments described above should be understood to be illustrative rather than restrictive in every respect. The scope of the present disclosure should be construed as the meaning and scope of the appended claims rather than the detailed description, and all changes or variations derived from the equivalent concepts fall within the scope of the present disclosure.

## Claims

1. A recombinant microorganism of the genus *Corynebacterium* having L-ornithine-producing ability, the recombinant microorganism comprising a LysE/ArgO family amino acid transporter protein derived from a strain of the genus *Shewanella* or a polynucleotide encoding the LysE/ArgO family amino acid transporter protein.

2. The microorganism of claim 1, wherein the protein comprises an amino acid sequence having 80% or more sequence identity to an amino acid sequence of SEQ ID NO: 1 or an amino acid sequence of SEQ ID NO: 3.

3. The microorganism of claim 1, wherein the strain of the genus *Shewanella* is a *Shewanella corallii* or *Shewanella oneidensis* strain.

4. The microorganism of claim 1, wherein activity of ornithine carbamoyltransferase subunit F is further weakened.

5. The microorganism of any one of claims 1 to 4, wherein activity of arginine repressor is further weakened.

6. The microorganism of claim 1, wherein the microorganism is *Corynebacterium glutamicum.*

7. A method of producing L-ornithine, the method comprising the step of culturing, in a medium, a recombinant microorganism of the genus *Corynebacterium* having L-ornithine-producing ability, the recombinant microorganism comprising a LysE/ArgO family amino acid transporter protein derived from a strain of the genus *Shewanella* or a polynucleotide encoding the LysE/ArgO family amino acid transporter protein.

8. The method of claim 7, wherein the protein comprises an amino acid sequence having 80% or more sequence identity to an amino acid sequence of SEQ ID NO: 1 or an amino acid sequence of SEQ ID NO: 3.

9. The method of claim 7, wherein the strain of the genus *Shewanella* is a *Shewanella corallii* or *Shewanella oneidensis* strain.

10. The method of claim 7, wherein activity of ornithine carbamoyltransferase subunit F in the microorganism is further weakened.

11. The method of any one of claims 7 to 10, wherein activity of arginine repressor in the microorganism is further weakened.

12. The method of claim 7, wherein the microorganism is *Corynebacterium glutamicum.*
